# EUROPEAN PATENT APPLICATION

(11) **EP 3 470 092 A1**
(43) Date of publication of application: **17.04.2019**
(21) Application number: 17813097.7
(22) Date of filing: 23.05.2017
(51) Int. Cl.: A61L 15/24

(54) **LIVESTOCK BODY PROTECTIVE MATERIAL, LIVESTOCK BODY PROTECTIVE FILM FORMING KIT, AND METHOD FOR PROTECTING LIVESTOCK'S SICK/INJURED AREA**

(30) Priority: 14.06.2016 JP 2016117683
(71) Applicant: Tokuyama Corporation, Shunan-shi, Yamaguchi 745-8648 (JP)
(72) Inventor: KONDO, Hitoshi, Shunan-shi Yamaguchi 745-8648 (JP); INUI, Yoji, Shunan-shi Yamaguchi 745-8648 (JP); WADA, Hiroshi, Nasushiobara-shi Tochigi 329-2801 (JP)
(74) Representative: Zoller, Markus
(86) International application number: PCT/JP2017/019173
(87) International publication number: WO 2017/217194

(57) **Abstract**

Provided is a livestock body protective material that includes (A) an emulsion obtained by dispersing a rubber-based macromolecule serving as a dispersoid in a dispersion medium including water as the main component, and (B) a coagulant blended according to need, and that is used to form a protective film for protecting a livestock's sick/injured area.

## Description

### TECHNICAL FIELD

The present invention relates to a novel livestock body protective material for forming a protective film for protection of a wound occurring in livestock, an operation scar (suture) of livestock, and an inflammation area of livestock or the like (hereinbelow, they are collectively described as "livestock's sick or injured area"). The present invention also relates to a novel kit for forming a livestock body protective film used to form the protective film, and a novel method for protecting a livestock's sick or injured area by using the livestock protective material and the kit for forming a livestock body protective film.

### BACKGROUND ART

Livestock often suffer from wounds as they hit ground surface or parts of a barn or the like. Furthermore, there are many cases in which, due to diseases or the like, a surgery is performed on livestock followed by suturing. For example, a disease named displaced abomasum easily occurs in dairy cow, and, in that case, it is necessary to cut and suture an abdominal area over a broad range of several tens of centimeters. Furthermore, livestock often have an occurrence of inflammation due to a disease or the like. For example, a dairy cow easily has an inflammation like foot fungus between toes.

With regard to a material for protecting the livestock's sick or injured area described above, an appropriate material is not present at current state. For example, it is very difficult to wrap a bandage around a livestock body due to movement of the livestock. Furthermore, if the sick or injured area is large or has a complex shape, there is a case in which a bandage may not be wrapped well around the area so that it cannot serve as a suitable protective material. Furthermore, even when the bandage can be wrapped around, there are many cases in which, in accordance with a contact of a bandage with ground surface or parts of a barn, the bandage is easily fallen off. Furthermore, there are also many cases in which, even when the bandage can be wrapped around, it is immediately fallen off from a moving part like knee of livestock.

Unlike humans, livestock have no idea of careful handling of a treatment area, and thus they are in a situation in which it is difficult to apply products for human use to them. Due to this reason, there is a case in which, depending on dairy farm, a method of fixing gauze on a livestock body with an aid of an adhesive is employed. However, this method not only requires lots of handwork but also is not desirable on livestock.

### DISCLOSURE OF THE INVENTION

### Problems to be Solved by the Invention

Thus, an object of the present invention is to provide a novel livestock body protective material for forming a protective film for protection of a livestock's sick or injured area, a novel kit for forming a livestock body protective film, and a novel method for protecting a livestock's sick or injured area by using the livestock protective material and the kit for forming a livestock body protective film.

### Means for Solving the Problems

Inventors of the present invention conducted intensive studies to solve the problem described above. As a result, the inventors found that the problem can be solved by using, as a material for protecting livestock's sick/injured area, an emulsion obtained by dispersing a rubber-based polymer serving as a dispersoid in a dispersion medium including water as the main component, and completed the present invention accordingly. Namely, the present invention includes the following aspects.
<1> A livestock body protective material including (A) an emulsion obtained by dispersing a rubber-based polymer serving as a dispersoid in a dispersion medium including water as a main component, and that is used to form a protective film for protecting a livestock's sick or injured area.
<2> The livestock body protective material described in <1>, further including (B) a coagulant.
<3> The livestock body protective material described in <2>, in which (B) the coagulant is at least one type selected from calcium salt and magnesium salt.
<4> The livestock body protective material described in <2>, in which (B) the coagulant is at least one type selected from the group consisting of lactic acid, citric acid, acetic acid, malic acid, fumaric acid, maleic acid, tartaric acid, gluconic acid, succinic acid, propionic acid, and butyric acid.
<5> The livestock body protective material described in any one of <1> to <4>, further including at least one type selected from the group consisting of (C) a surfactant, (D) a sterilizing disinfectant, and (E) a pharmaceutical component.
<6> A kit for forming a livestock body protective film that is composed of a combination of (i) a base containing (A) an emulsion obtained by dispersing a rubber-based polymer serving as a dispersoid in a dispersion medium including water as a main component, stored in a predetermined container, and (ii) a curing agent including (B) a coagulant, stored in a container different from the above container, and that is used to form a protective film for protecting a livestock's sick or injured area.
<7> The kit for forming a livestock body protective film described in <6>, in which (ii) the curing agent further contains (F) water and/or a water-soluble solvent.
<8> A method for protecting a livestock's sick or injured area including forming a protective film for protection of a livestock's sick or injured area by adhering the livestock body protective material described in any one of <1> to <5> on a livestock's sick or injured area.
<9> A method for protecting a livestock's sick or injured area including forming a protective film for protection of a livestock's sick or injured area by adhering any one of (i) a base containing (A) an emulsion obtained by dispersing a rubber-based polymer serving as a dispersoid in a dispersion medium including water as a main component and (ii) a curing agent including (B) a coagulant on a livestock's sick or injured area, and then adhering the other on the livestock's sick or injured area.
<10> Use of the livestock body protective material described in any one of <1> to <5> or the kit for forming a livestock body protective film described in <6> or <7> for forming a protective film for protection of a livestock's sick or injured area.

### Effects of the Invention

According to the present invention, a novel livestock body protective material for forming a protective film for protection of a livestock's sick or injured area, a kit for forming a livestock body protective film, and a novel method for protecting a livestock's sick or injured area by using the livestock protective material and the kit for forming a livestock body protective film can be provided.

### PREFERRED MODE FOR CARRYING OUT THE INVENTION

The livestock body protective material of the present disclosure includes (A) an emulsion obtained by dispersing a rubber-based polymer serving as a dispersoid in a dispersion medium including water as a main component (hereinbelow, also simply described as "(A) the emulsion"), and it is used for forming a protective film for protection of a livestock's sick or injured area.

Herein, the livestock of the present disclosure is not particularly limited, and examples thereof include a cow, a horse, a pig, a sheep, a goat, a dog, and a cat.

The livestock body protective material of the present disclosure exhibits very high adhesiveness to skin or the like of livestock. Furthermore, as an emulsion (liquid form) is used, a protective film can be easily formed regardless of the shape or size of a sick or injured area. Furthermore, when the livestock body protective material of the present disclosure is adhered to a livestock's sick or injured area, a protective film is formed within a short time, and thus the operability (workability) is excellent. Furthermore, although a protective film is immediately formed when the livestock body protective material of the present disclosure is adhered to a livestock's sick or injured area, the protective film turns into a perfect protective film having high adhesiveness and strength according to natural drying for about 5 to 10 minutes, for example.

Furthermore, since an emulsion having water as a main component of the dispersion medium is used, it might be also speculated that the livestock body protective material of the present disclosure does not tightly adhere on hair of livestock. However, it can actually form a protective film which exhibits high adhesiveness even on the hair. In this regard, that is also presumably caused by an action of a rubber-based polymer, which is a dispersoid of the emulsion. As a result, it is not necessary to cut the hair near a sick or injured area more than needed, and thus desirable in terms of the operability (workability).

Since a region near the sick or injured area is sealed by forming a protective film as described above, pathogen's intrusion to a sick or injured area is prevented so that the sick or injured area can be healed all the while. Furthermore, since the protective film is formed of a rubber-based polymer, even when the sick or injured area is brought into contact with ground surface or the like or the protective film is stretched or the like due to a change in livestock's posture, it is hardly fallen off from the sick or injured area due to excellent shape followability. Furthermore, according to the livestock body protective material of the present disclosure, a protective film with uniform thickness can be easily formed, and thus, even when a high load is applied to the protective film as livestock take a rest in side-lying position or the like, it is hardly fallen off or broken or the like, and thus protection of a sick or injured area over a long period of time can be achieved. In addition, by human hands, the protective film can be peeled within a short time.

Hereinbelow, each constitutional component of the livestock body protective material of the present disclosure is described in detail.

### [(A) Emulsion]

The livestock body protective material of the present disclosure includes an emulsion obtained by dispersing a rubber-based polymer serving as a dispersoid in a dispersion medium including water as a main component.

The dispersion medium of (A) the emulsion contains water as a main component, but it is also possible to contain a small amount of an organic solvent in addition to water. For example, it is possible that the dispersion medium contains an organic solvent with microbicidal activity like ethanol at a ratio of 0.1 to 10% by mass. Content ratio of an organic solvent in the dispersion medium is preferably 10% by mass or less, more preferably 5% by mass or less, and even more preferably 0% by mass.

Examples of the rubber-based polymer as a dispersoid of (A) the emulsion include natural rubber; synthetic rubber such as isoprene rubber, butyl rubber, butadiene rubber, ethylene rubber, ethylene-butadiene rubber, propylene rubber, ethylenepropylene rubber, styrene rubber, styrene-butadiene rubber, styrene-isoprene rubber, urethane rubber, thiokol rubber, nitrile rubber, nitrile-butadiene rubber, chloroprene rubber, chlorosulfonated polyethylene rubber, chlorinated polyethylene rubber, acrylic rubber, vinyl acetate rubber, ethylene-vinyl acetate rubber, epichlorohydrin rubber, silicone rubber, polysulfide rubber, polyether rubber, fluoro rubber, or polysulfide rubber; or the like.

Furthermore, as for the rubber-based polymer, derivatives of natural rubber or synthetic rubber can be also used. Examples of the derivatives of natural rubber or synthetic rubber include those obtained by copolymerizing monomers which constitute natural rubber or synthetic rubber and those obtained by modifying the surface of natural rubber or synthetic rubber with a reactive functional group having functionality such as carboxy group, amino group, or trimethylsilyl group. Furthermore, the form of the copolymerization may be any of random copolymerization, alternating copolymerization, periodic copolymerization, block copolymerization, and graft copolymerization.

Among the rubber-based polymers that are described above, natural rubber, isoprene rubber, urethane rubber, butadiene rubber, nitrile rubber, chloroprene rubber, and vinyl acetate rubber are preferable from the viewpoint that the protective film to be obtained has high elongation and high strength. Among them, those in which a part of them has been crosslinked in advance have even higher strength, and thus are more preferable.

The rubber-based polymer can be a mixture of two or more kinds thereof. Structure of the rubber-based polymer can be any of linear, branched, dendrimeric, networked, cyclic, or the like.

As for (A) the emulsion, those containing various stabilizers such as ammonia and surfactants such as glycerin fatty acid ester or lauric acid ester in order for the rubber-based polymer to form stable particles in the emulsion can be used. Furthermore, as for (A) the emulsion, in accordance with need, those added with a vulcanizing agent, a vulcanization accelerator, a vulcanization acceleration aid, a vulcanization retardant, a deterioration inhibitor (antioxidant, ozone inhibitor, or the like), processing aids (plasticizers, softeners, tackifiers, or the like), dispersants, creaming agents, foaming agents, heat-sensitive agents (zinc ammonium complex salts, polyvinyl methyl ether, or the like), or the like may be used.

The solid component concentration in (A) the emulsion is not particularly limited. The solid component concentration in (A) the emulsion is preferably 20 to 90% by mass, for example, and more preferably 40 to 85% by mass. As the solid component concentration in (A) the emulsion is 20% by mass or more, there is a tendency that the time required for forming a protective film is further shortened and the strength of a protective film further increases. On the other hand, as the solid component concentration in (A) the emulsion is 90% by mass or less, there is tendency that the applicability on a sick or injured area is improved since an increase in the viscosity of the livestock body protective material is suppressed.

Furthermore, the solid component concentration in (A) the emulsion can be obtained by drying the livestock body protective material. However, when the livestock body protective material includes an inorganic material, the inorganic material will be included in a dried product that is obtained simply by drying. In that case, by carrying out first an analysis of a dried product obtained by drying the livestock body protective material, components included in the dried product are analyzed in advance. Then, according to combustion of the dried product, a blending amount of the inorganic material can be obtained from a mass of the residuals that are obtained after the combustion. Furthermore, the amount of the solid content (rubber-based polymer) included in the emulsion can be obtained from the combusted mass. According to this method, the solid component concentration in the emulsion can be obtained also from the livestock body protective material.

### [(B) Coagulant]

To enable forming of a protective film having excellent adhesiveness within even shorter time, the livestock body protective material of the present disclosure preferably contains (B) a coagulant. In that case, it is possible that the livestock body protective material is prepared and stored as one composition by blending (A) the emulsion with (B) the coagulant, or, as it is described below, it is also possible that (A) the emulsion (component of a base) and (B) the coagulant (component of a curing agent) are contained in separate containers and stored as a kit for forming a livestock body protective film. By storing separately the base and curing agent like that, the storage stability can be enhanced.
In (A) the emulsion, the surface of polymer fine particles constituting the rubber-based polymer exhibits a negative charge as hydrophilic groups of an emulsifying agent molecule (sulfonic acid group, hydroxy group, or the like) bind thereto, for example. (B) The coagulant neutralizes charges on a surface of polymer fine particles constituting the rubber-based polymer, and, consequently, has a role of coagulating the rubber-based macromolecule, which is an aggregate of polymer fine particles.
(B) The coagulant is not particularly limited, and examples thereof include a low molecular inorganic coagulant, a polymer inorganic coagulant, a polymer organic coagulant, and an acid. The coagulant may be used either singly or used as a combination of two or more kinds thereof.

### (Low molecular inorganic coagulant (metal salt)

The low molecular inorganic coagulant is exemplified by a wide variety of metal salts. Among the coagulants, specifically, the metal salt effectively causes the coagulation of rubber-based polymer exhibiting a negative charge. The metal salt functioning as the coagulant is defined in a broad sense as the compound in which an anion derived from an acid and a cation derived from a base are ionically bonded, and it is a compound generated by a neutralization reaction between an acid and a base, a reaction between an acid and a basic oxide, a reaction between an acid and a metal simple substance, a reaction between a base and an acidic oxide, a reaction between a base and a nonmetal simple substance, a reaction between an acidic oxide and a basic oxide, and a reaction between a nonmetal simple substance and a metal, and is capable of being ionized in the dispersion medium of an emulsion to produce metal ions. Type of the metal salt is not particularly limited as long as it has the aforementioned function, and all of the well-known metal salts can be used. Examples of the metal salt include calcium chloride, magnesium chloride, iron chloride, tin chloride, aluminum chloride, titanium chloride, sodium chloride, potassium chloride, calcium sulfate, magnesium sulfate, zinc sulfate, aluminum sulfate, iron sulfate, zirconium sulfate, sodium sulfate, potassium sulfate, zinc oxide, magnesium oxide, aluminum oxide, iron oxide, titanium oxide, zirconium oxide, tin oxide, aluminum hydroxide, iron hydroxide, zirconium hydroxide, tin hydroxide, sodium hydroxide, potassium hydroxide, zinc acetate, sodium acetate, potassium acetate, calcium lactate, zinc lactate, sodium lactate, potassium lactate, and potassium fluorotitanate. The metal salts may be a mixture of two or more kinds thereof. Among them, from the viewpoint of coagulating rapidly the rubber-based polymer, a divalent metal salt is preferable, and at least one type selected from calcium salt and magnesium salt is more preferable.

### (Polymer inorganic coagulant)

As for the polymer inorganic coagulant, poly-aluminum chloride ([Al₂ (OH)ₙCl₆₋ₙ]ₘ), poly-aluminum sulfate ([Al₂(OH)ₙ(SO₄)_{3-n/2}]ₘ), poly-iron (III) chloride ([Fe₂(OH)ₙCl₆₋ₙ]ₘ), poly-iron (III) sulfate ([Fe₂(OH)ₙ(SO₄)_{3-n/2}]ₘ), or the like may be exemplified. The polymer inorganic coagulant may be a mixture of two or more kinds thereof.

### (Polymer organic coagulant)

As for the polymer organic coagulant, polyacrylamide, polyethylene oxide, urea-formalin resin, and the like are exemplified as the nonionic coagulant, sodium polyacrylate (acrylamide-sodium acrylate copolymer), a polyacrylamide partial hydrolyzate, sulfomethylated polyacrylamide, polyaminoalkyl(meth)acrylate, halogenated polyvinyl pyridium, halogenated polydiallyl ammonium, and the like are exemplified as the anionic coagulant, and polyamino methyl acrylamide, polyvinyl imidazoline, chitosan, epoxyamine, and the like are exemplified as the cationic coagulant. These coagulants have molecular weights from several tens of thousands to several millions, and the greater the molecular weight, the higher the charge becomes. The polymer organic coagulant may be a mixture of two or more kinds thereof.

### (Acid)

The acid is not particularly limited. From the viewpoint of having safety and coagulating rapidly the rubber-based polymer, at least one type selected from the group consisting of lactic acid, citric acid, acetic acid, malic acid, fumaric acid, maleic acid, tartaric acid, gluconic acid, succinic acid, propionic acid, and butyric acid is preferable. The acid may be a salt with metal or a mixture of two or more kinds thereof.

When the livestock body protective material of the present disclosure contains (B) the coagulant, content of (B) the coagulant is, from the viewpoint of forming a protective film within a short time, preferably 2 to 300 parts by mass, and more preferably 5 to 200 parts by mass relative to 100 parts by mass of (A) the emulsion. As the content of (B) the coagulant is set at 2 parts by mass or more, the rubber-based polymer tends to coagulate more rapidly. On the other hand, as the content of (B) the coagulant is set at 300 parts by mass or less, the amount of the rubber-based polymer becomes more sufficient, and thus a protective film with more sufficient film thickness tends to form.

### (Storage form or the like)

When the livestock body protective material of the present disclosure contains (B) the coagulant, a protective film can be formed according to adhesion of the livestock body protective material including (A) the emulsion and (B) the coagulant onto a livestock's sick or injured area. However, to have more time for operation and to enhance more the operability, it is preferable to have form in which the storage is made in two separate pieces, that is, a kit for forming a livestock body protective film which is composed of a combination of (i) a base containing (A) the emulsion (hereinbelow, also simply described as "(i) the base") stored in a predetermined container, and (ii) a curing agent including (B) the coagulant (hereinbelow, also simply described as "(ii) the curing agent") that is stored in a container different from the aforementioned container.

When the storage is made as a kit for forming a livestock body protective film like the one described above, (ii) the curing agent preferably contains (F) water and/or a water-soluble solvent from the viewpoint of further enhancing the adhesiveness of a protective film to be formed.

As for the water-soluble solvent, it is not particularly limited as long as it can sufficiently dissolve (B) the coagulant and is not so significantly harmful to humans or livestock. In particular, ethanol, propanol, butanol, acetone, or the like is preferable. However, since there is a case in which the water-soluble solvent exhibits an undesirable influence on humans or livestock, use amount thereof is preferably 0 to 50 parts by mass, and more preferably 0 to 10 parts by mass relative to 100 parts by mass of (B) the coagulant. Furthermore, when water is used, use amount of water is not particularly limited, and it is preferably 150 to 4000 parts by mass, and more preferably 200 to 3000 parts by mass relative to 100 parts by mass of (B) the coagulant. As the use amount of water is set at 150 parts by mass or more, (B) the coagulant tends to get dissolved sufficiently in water. On the other hand, as the use amount of water is set at 4000 parts by mass or less, the rubber-based polymer tends to coagulate rapidly.

Hereinabove, descriptions are given for the constitution of the livestock body protective material of the present disclosure and storage form (kit) that is suitable for a case of including (B) the coagulant, but, depending on necessity, the livestock body protective material of the present disclosure may include other various additives. Examples of the additives include (C) a surfactant, (D) a sterilizing disinfectant, (E) a pharmaceutical component, a filler, a thickening agent, and a coloring agent. Among them, at least one type selected from the group consisting of (C) the surfactant, (D) the sterilizing disinfectant, and (E) the pharmaceutical component is preferable.

### [(C) Surfactant]

The livestock body protective material of the present disclosure may include (C) the surfactant. As the livestock body protective material includes (C) the surfactant, not only the rubber-based polymer is stabilized within the dispersion medium, but there is also the effect of having better affinity to a sick or injured area covered with oil and fat, and the applicability of the livestock body protective material tends to improve. Furthermore, as the livestock body protective material includes (C) the surfactant, the tack property of the protective film on a sick or injured area can be adjusted. Furthermore, as the livestock body protective material includes (C) the surfactant, the protective film tends to get peeled more smoothly. It is considered that, as a result, wild behavior of livestock at the time of peeling the protective film can be suppressed and damage in a sick or injured area can be also suppressed at the time of peeling the protective film.
(C) The surfactant is not particularly limited, and it can be any of anionic surfactants, cationic surfactants, amphoteric surfactants, and nonionic surfactants. These surfactants may be used either singly or a combination of two or more kinds thereof may be used.

Examples of the anionic surfactant include alkyl carboxylate, alkyl sulfate, alkylsulfonate, alkylbenzene sulfonate, and alkyl ether carboxylate. Among them, alkyl carboxylate, alkyl sulfate, and alkylsulfonate in which the alkyl group has carbon atom number of 6 to 18 are preferable, and ammonium laurate, sodium laurate, sodium lauryl sulfate, and sodium dodecane sulfonate are more preferable.

Examples of the cationic surfactant include alkylamine salt and quaternary ammonium salt. Among them, quaternary ammonium salt is preferable, and tetrapropylammonium chloride or the like is more preferable.

Examples of the amphoteric surfactant include aminocarboxylate.

Examples of the nonionic surfactant include polyoxyethylene alkyl ether, polyoxyethylene alkylphenyl ether, polyoxyethylene-polyoxypropylene block polymer, glycerin fatty acid esters, polyglycerin fatty acid esters, polyoxyethyleneglycerin fatty acid esters, polyoxyglycerin fatty acid esters, sorbitan fatty acid ester, sucrose ester, polyoxydiethylene alkylamine, and block polymers of polyoxysiloxanes and polyoxyethylenes. Examples of the polyglycerin fatty acid ester include those obtained by the esterification reaction of polyglycerin and fatty acid at a ratio of 1 : 1 to 1 : 4 by a conventional method, in which it contains as a main component the compound in which part of the hydroxy groups contained in polyglycerin form an ester bonded with the fatty acid. Examples of the polyglycerin include polyglycerin having a degree of polymerization of n = 1 to 10 such as monoglycerin, diglycerin, triglycerin, tetraglycerin, or decaglycerin. Furthermore, examples of the fatty acid include those with carbon atom number of 8 to 18 such as lauric acid, myristic acid, stearic acid, or oleic acid. Examples of such polyglycerin fatty acid ester include decaglyceryl trioleate.

Among those surfactants, from the viewpoint of further enhancing the applicability to a sick or injured area covered with oil and fat, an anionic surfactant is preferable. The reason for having enhanced applicability of the livestock body protective material by an anionic surfactant is, although not clear enough, considered as follows; there are many cases in which the surface of rubber-based polymer is anionically (negatively) charged, and in that case, if a cationic surfactant is used, there is a case in which the deterioration of the applicability resulting from poor dispersion of an emulsion including the rubber-based polymer occurs due to the electrostatic interaction. On the other hand, in a case of using an anionic surfactant, it is believed that such poor dispersion is not likely to occur.

In a case in which the livestock body protective material of the present disclosure includes (C) the surfactant, content of (C) the surfactant is preferably 0.05 to 10% by mass, and more preferably 0.10 to 5% by mass relative to the entire livestock body protective material, from the viewpoint of the applicability on a sick or injured area covered with oil and fat and the strength of the protective film to be formed. As the content of (C) the surfactant is set at 0.05% by mass or more, the applicability on a sick or injured area covered with oil and fat tends to get enhanced more. On the other hand, as the content of (C) the surfactant is set at 10% by mass or less, the strength of a protective film to be formed tends to increase more.

Furthermore, in a case in which the livestock body protective material of the present disclosure includes (B) the coagulant and the storage is made such that (i) the base and (ii) the curing agent are present separately, (C) the surfactant can be added to any one of (i) the base and (ii) the curing agent, but it is preferably added to (i) the base. In that case, content of (C) the surfactant is preferably 0.05 to 10% by mass, and more preferably 0.10 to 5% by mass relative total of (i) the base.

### [(D) Sterilizing disinfectant]

The livestock body protective material of the present disclosure may also include (D) a sterilizing disinfectant. As the livestock body protective material includes (D) the sterilizing disinfectant, an increase in the number of bacteria in a sick or injured area can be suppressed.
(D) The sterilizing disinfectant is not particularly limited as long as the sterilizing disinfectant can kill harmful microbes such as bacteria, fungi, and viruses which cause diseases, and examples thereof include an iodine compound, metals such as silver, copper, zinc, titanium, and iron and metal salts, tea leaf powder, hinoki cypress powder, chitosan, benzalkonium chloride, benzethonium chloride, fatty acid ester such as caprylic acid monoglyceride, triclosan, isopropylmethylphenol, cetylpyridinium chloride, resorcin, trichlorocarbanide, halocarbon, chlorhexidine, chlorhexidine hydrochloide, chlorhexidine gluconate, acrinol, sodium hypochlorite, and hydrogen peroxide.

Among them, an iodine compound and silver are preferable from the viewpoints of skin irritation to a human or livestock, sustainability of the sterilizing and disinfecting effect, and cost. Examples of the iodine compound include iodine, povidone iodine, sodium iodate, potassium iodate, sodium iodide, potassium iodide, and iodoform.

In a case in which the livestock body protective material of the present disclosure includes (D) the sterilizing disinfectant, content of (D) the sterilizing disinfectant is preferably 0.1 to 10% by mass relative to the entire livestock body protective material.

Furthermore, in a case in which the livestock body protective material of the present disclosure includes (B) the coagulant and also the storage is made such that (i) the base and (ii) the curing agent are present separately, (D) the sterilizing disinfectant can be added to any one of (i) the base and (ii) the curing agent, or added to both of them.

### [(E) Pharmaceutical component]

The livestock body protective material of the present disclosure may also contain (E) a pharmaceutical component. As the livestock body protective material includes (E) the pharmaceutical component, rapid healing of a sick or injured area can be achieved. Examples of (E) the pharmaceutical component include an extract of aloe vera leaves and eucalyptus oil.

In a case in which the livestock body protective material of the present disclosure includes (E) the pharmaceutical component, content of (E) the pharmaceutical component is preferably 0.1 to 10% by mass relative to the entire livestock body protective material.

Furthermore, in a case in which the livestock body protective material of the present disclosure includes (B) the coagulant and also the storage is made such that (i) the base and (ii) the curing agent are present separately, (E) the pharmaceutical component can be added to any one of (i) the base and (ii) the curing agent, or added to both of them.

### [Filler]

The livestock body protective material of the present disclosure may include a filler to adjust physical properties of a protective film to be formed. Examples of the filler include oxides of metals or semimetals such as silica and alumina, and inorganic fluorine compounds such as potassium fluorotitanate or potassium fluorosilicate.

In a case in which the livestock body protective material of the present disclosure includes a filler, content of the filler is preferably 0.5 to 30% by mass relative to the entire livestock body protective material.

Furthermore, in a case in which the livestock body protective material of the present disclosure includes (B) the coagulant and also the storage is made such that (i) the base and (ii) the curing agent are present separately, the filler can be added to any one of (i) the base and (ii) the curing agent, or added to both of them.

### [Thickening agent]

The livestock body protective material of the present disclosure may include a thickening agent in order to adjust the viscosity and to improve the yield of a protective film. Examples of the thickening agent include an inorganic thickening agent and a water-soluble polymer. The inorganic thickening agent is generally a compound such as colloidal magnesium aluminum silicate or colloidal clay, and they are fumed or precipitated to make particles having a high surface-size ratio. Examples of the water-soluble polymer include those obtained by polymerizing a monomer such as vinyl alcohol, vinyl pyrrolidone, methyl vinyl ether, acrylic acid, methacrylic acid, acrylamide, ethylene oxide, or ethyleneimine.

Furthermore, in a case in which the livestock body protective material of the present disclosure includes (B) the coagulant and also the storage is made such that (i) the base and (ii) the curing agent are present separately, the thickening agent can be added to any one of (i) the base and (ii) the curing agent, or added to both of them.

### [Coloring agent]

The livestock body protective material of the present disclosure may include a coloring agent in order to have coloration of a protective film to be formed and to enhance the visibility of a protective film. Examples of the coloring agent include titania particles, calcium carbonate, and zinc oxide, and titania particles are preferable among them.

Furthermore, in a case in which the livestock body protective material of the present disclosure includes (B) the coagulant and also the storage is made such that (i) the base and (ii) the curing agent are present separately, the coloring agent can be added to any one of (i) the base and (ii) the curing agent, or added to both of them.

### [Other additives]

The livestock body protective material of the present disclosure may include additives other than those described above within a range in which the effect of the present invention is not impaired. For example, in order to improve skin roughness or erosion, the livestock body protective material of the present disclosure may also include a moisturizing component or a cosmetic compound. Examples of the moisturizing component include sodium hyaluronic acid, glycerin, lanolin, and sorbitol. Examples of the cosmetic component include castor oil and potassium palm fatty acid. Furthermore, the livestock body protective material of the present disclosure may include a repellent component in order to prevent approach of insects or the like to a protective film, and also may include a bitter-taste component to prevent wrongful recognition of a protective film by livestock.

Furthermore, in a case in which the livestock body protective material of the present disclosure includes (B) the coagulant and also the storage is made such that (i) the base and (ii) the curing agent are present separately, the additives can be added to any one of (i) the base and (ii) the curing agent, or added to both of them.

### [Method for production and method for use]

### (Method for producing livestock body protective material)

Method for producing the livestock body protective material of the present disclosure (or, each of (i) the base and (ii) the curing agent for a case in which the storage is made such that (i) the base and (ii) the curing agent are present separately) is not particularly limited, but the production can be made by using a known stirring mixer. Examples of the stirring mixer include a rotating container type mixing kneader such as a ball mill; a fixed container type mixing kneader having a horizontal axis or a vertical axis such as a ribbon mixer, a co-kneader, an internal mixer, a screw kneader, a Henschel mixer, a universal mixer, a Loedige mixer, a butterfly mixer, or a blade stirring device; and the like.

Furthermore, in the production of (ii) the curing agent, when (B) the coagulant and the like has high solubility in (F) water and/or the water-soluble solvent, a stirring device by which no strong shearing force is applied to the component as a subject for dissolution or a solution having the component dissolved therein may be used. Examples of the stirring device include a portable stirrer, a vertical stirrer, a side entering stirrer, an inline stirrer, and a blade stirring device, equipped with various stirring wings.

### (Method for forming protective film)

According to adhesion of the livestock body protective material of the present disclosure onto a livestock's sick or injured area, a protective film for protecting the sick/injured area can be formed. By forming the protective film, contact of a sick or injured area with dirts or excretions from outside can be prevented.

In a case in which the livestock body protective material of the present disclosure includes (B) the coagulant and also the storage is made such that (i) the base and (ii) the curing agent are present separately, it is also possible that the protective film is formed after preparing, immediately before use, the livestock body protective material by mixing (i) the base and (ii) the curing agent. Alternatively, it is also possible that one of (i) the base and (ii) the curing agent is adhered on a sick or injured area, and then the other is adhered on the sick or injured area so as to form a protective film. The adhesion order is not particularly limited, but, to form a uniform protective film even in a sick or injured area covered with oil and fat, it is preferable that (i) the base is adhered first, and then (ii) the curing agent is rapidly adhered.

For forming a protective film, it is also possible that, after applying a pharmaceutical preparation to a sick or injured area, the protective film is formed according to the above method. Accordingly, the pharmaceutical preparation can be placed on a sick or injured area, and, at the same time, contact of a sick or injured area with dirts or excretions from outside can be prevented. Furthermore, for forming a protective film, it is also possible to form a protective film so as to have larger area than a material for application, like covering the material for application after placing the material for application like gauze on a sick or injured area. By tightly adhering at least part of the protective film on skin or the like of livestock, the material for application can be fixed, and, at the same time, contact of a sick or injured area with dirts or excretions from outside can be prevented.

Method for adhering the livestock body protective material (or, each of (i) the base and (ii) the curing agent for a case in which (i) the base and (ii) the curing agent are separately adhered) is not particularly limited, and examples thereof include a spray method, a brush application method, and a dipping method. Among them, from the viewpoint that the protective film can be conveniently formed regardless of the shape or size of a sick or injured area, the spray method and brush application method are preferable and the spray method is more preferable.

In a case in which the spray method is used, viscosity of the livestock body protective material (or, each of (i) the base and (ii) the curing agent for a case in which (i) the base and (ii) the curing agent are separately adhered) is preferably 0.1 to 50 dPa·s from the viewpoint of the easiness of spraying.

In a case in which (i) the base and (ii) the curing agent are separately adhered, mass ratio of the adhesion amount on a sick or injured area ((i) the base/(ii) the curing agent) is generally within a range of from 0.5 to 2, although it is not particularly limited thereto.

Furthermore, in a case in which only (i) the base is used, the blending ratio of each component to be included in the livestock body protective material has the same value as the amount ratio of the component to be included in (i) the base prepared. In a case in which (i) the base and (ii) the curing agent are separately adhered, use level of (i) the base and (ii) the curing agent and nature of operation thereof are determined in advance with regard to an actual method like spray method, and, from the use amount thereof and the amount ratio of each component to be included in (i) the base and (ii) the curing agent, the blending ratio of each component to be included in the livestock body protective material can be obtained.

### (Protective film)

The protective film formed of the livestock body protective material of the present disclosure is used for protection of a livestock's sick or injured area. Since this protective film exhibits very favorable adhesiveness even when body hair or the like is present, it can be tightly adhered to a sick or injured area for a long period of time without being fallen off. Furthermore, since the protective film has a rubber-based polymer as a main component, it has excellent shape followability, and is unlikely to be fallen off even when a sick or injured area is present in a moving part. Furthermore, the protective film can be easily peeled and it hardly has a problem of removing skin or the like at the time of peeling like a case of using an adhesive.

### EXAMPLES

Hereinbelow, the livestock body protective material of the present disclosure is specifically described in view of Examples and Comparative Examples, but the present invention is not limited to those examples.

In Examples and Comparative Examples, raw materials that are used for production of a livestock body protective material and abbreviations thereof, and the method for evaluating "operability" (time for application work, time for peeling work, and time evaluation) and "safety" (skin peeling from wound area, number of bacteria) are as described below.

### <Raw materials>

### (A) Emulsion

Natural rubber emulsion: NR (solid component concentration: 50% by mass, viscosity: 50 mPa·s, minimum film-forming temperature: 10°C, dispersion medium: water)
Isoprene rubber emulsion: IR (solid component concentration: 50% by mass, viscosity: 50 mPa·s, minimum film-forming temperature: 10°C, dispersion medium: water)

### (B) Coagulant

Lactic acid: LA
Calcium chloride: CC

### (C) Surfactant

Ammonium lauric acid: LAA

### (D) Sterilizing disinfectant

Povidone iodine: PVPI

### (E) Pharmaceutical component

Aloe extract: AR

### (F) Water and/or water-soluble solvent

Water: WA
Ethanol: ET

### <(i) Base and (ii) curing agent>

(i) Base: Blending amount of a base containing each raw material described above is shown in Table 1 (unit: parts by mass).

**[Table 1]**

| Composition | (A) Emulsion | | (C) Surfactant | (D) Sterilizing disinfectant | (E) Pharmaceutical component |
|---|---|---|---|---|---|
| | NR | IR | LAA | PVPI | AR |
| (i)-1 | 100 | | | | |
| (i)-2 | | 100 | | | |
| (i)-3 | 99.5 | | 0.5 | | |
| (i)-4 | 99.5 | | | 0.5 | |
| (i)-5 | 97 | | | | 3 |

(ii) Curing agent: Blending amount of a curing agent containing (B) the coagulant is shown in Table 2 (unit: parts by mass).

**[Table 2]**

| Composition | (B) Coagulant | | (F) Water and/or water-soluble solvent | |
|---|---|---|---|---|
| | LA | CC | WA (Water) | ET |
| (ii)-1 | 100 | | | |
| (ii)-2 | | 100 | 500 | |
| (ii)-3 | | 100 | 500 | 30 |

(ii)-1 is a curing agent with composition in which (B) the coagulant (LA: lactic acid) is 100 parts by mass. (ii)-2 is a curing agent with composition in which 500 parts by mass of a solvent (WA: water) is included with 100 parts by mass of (B) the coagulant (CC: calcium chloride). (ii)-3 is a curing agent with composition in which 500 parts by mass of a solvent (WA: water) and 30 parts of a solvent (ET: ethanol) are included with 100 parts by mass of (B) the coagulant (CC: calcium chloride).

### <Method for evaluation>

### (1) Method for evaluating "operability"

### (Time for application work)

By using the livestock body protective materials of Examples 1 to 12, evaluation was made while having the time required for protecting a livestock's sick or injured area or a healthy area as time for application work. In Example 6 in which a gauze was applied as an application material, the evaluation was made by having the work time including the time for applying a gauze as the time for application work. Shorter time for application work indicates easier forming of a protective film. In Comparative Example 1, the time required for protection by wrapping a bandage around a livestock's sick or injured area or a healthy area was taken as the time for application work. In addition, a case in which the time for application work is 3 minutes or shorter is evaluated "A" and a case in which the time for application work is longer than 3 minutes is evaluated "C" (Time evaluation 1).

### (Time for peeling work)

One day after performing the above application work, the time required for complete peeling of the protective film was evaluated as the time for peeling work. Shorter time for peeling work indicates higher operability. In Comparative Examples, evaluation was made by having the time at which the bandage is removed as the time for peeling work. In addition, a case in which the time for peeling work is 3 minutes or shorter is evaluated "A" and a case in which the time for peeling work is longer than 3 minutes is evaluated "C" (Time evaluation 2).

### (2) Method for evaluating "safety"

### (Skin removal)

Presence or absence of skin removal at the time of peeling a protective film was observed with a naked eye. In addition, a case in which no skin removal has been confirmed is evaluated "A" and a case in which skin removal has been confirmed is evaluated "C".

### (Evaluation of number of bacteria)

The effect of blocking bacteria by a protective film was evaluated as follows. In Examples 10 to 12, the healthy area (about 30 cm × 30 cm) of livestock (dairy cow) was cleaned completely with alcohol, and a protective film composed of the livestock body protective material was formed on that part. The livestock was allowed to move freely for one day. After that, the protective film was peeled, and the part (about 1 cm²), to which the protective film had been formed before, was wiped with a cotton swab (Promedia ST-25 manufactured by ELMEX), and the cotton swab was dipped in 10 mL of physiological saline. In Comparative Example 1, the healthy area of livestock (dairy cow) was cleaned with alcohol, and then wrapped with a bandage. The livestock was then allowed to move freely for one day. After that, the bandage was removed, and the part (about 1 cm²), around which the bandage had been wrapped before, was wiped with a cotton swab (Promedia ST-25 manufactured by ELMEX), and the cotton swab was dipped in 10 mL of physiological saline.

1 mL of the physiological saline in which the cotton swab was dipped was added dropwise in a culture medium (aerobic bacteria culture, 6400AC, manufactured by 3M), and then cultured at 37°C for 48 hours. After culturing, the number of colonies in the culture medium was counted, and, by assuming that one bacteria forms one colony, the number of bacteria in the aforementioned 10 mL of physiological saline was calculated from the number of colonies (number of colonies × 10 = number of bacteria). The number of bacteria which has been obtained was evaluated according to the following evaluation criteria. Generally, if the number of bacteria is less than 1000/cm², it was deemed that cleanliness is maintained. Furthermore, the number of external bacteria (number of bacteria in soil) was roughly 10000/cm² or more although there is variation.

### - Evaluation criteria -

A: Less than 1000/cm² (The number of bacteria was low, and the protective film part was maintained in a clean condition.)
B: 1000/cm² or more to less than 10000/cm² (The number of bacteria was somewhat high, but it was less than the number of bacteria in the outside.)
C: 10000/cm² or more (The number of bacteria was high, so that the effect of reducing the number of bacteria by the protective film could not be confirmed.)

### [Example 1]

The base (i) -1 was directly used as a livestock body protective material. 50 g of the livestock body protective material (the base (i)-1) was directly applied by spraying to a sick or injured area (about 10 cm × 10 cm) of livestock (dairy cow) to form a protective film, and evaluation was made in terms of the "operability" and "safety". In Table 3, the blending amount of each component is shown, and the results of the operability and safety are shown in Table 4.

### [Examples 2 to 5]

The protective film was formed in the same manner as Example 1 except that only the bases (i)-2 to (i)-5 were used as a livestock body protective material, and evaluation was made in terms of the "operability" and "safety". In Table 3, the blending amount of each component is shown, and the results of the operability and safety are shown in Table 4.

### [Example 6]

First, a gauze was applied to a sick or injured area (about 10 cm × 10 cm) of livestock (dairy cow). Subsequently, 75 g of the livestock body protective material (the base (i)-1) was directly applied by spraying on top of the gauze to form a protective film. At that time, the area sprayed with the livestock body protective material had a slightly larger area (about 12 cm × 12 cm) than the gauze such that the livestock body protective material completely covered the gauze. In addition, evaluation was made in terms of the "operability" and "safety", in the same manner as Example 1. In Table 3, the blending amount of each component and the like are shown, and the results are shown in Table 4.

### [Example 7]

50 g of the base (i) -1 was directly applied by spraying on a sick or injured area (about 10 cm × 10 cm) of livestock (dairy cow). Subsequently, 50 g of a curing agent ((ii)-1) was applied by spraying on the part to which the base had been sprayed, and thus a protective film was formed. In Table 3, the blending amount of each component and the like are shown, and the results are shown in Table 4.

### [Examples 8 and 9]

To a sick or injured area of livestock (dairy cow), (i) the base and (ii) the curing agent were sprayed in this order in the same manner as Example 7, except that the composition of the livestock body protective material was as those described in Table 3. In addition, similarly to Example 7, evaluation was made in terms of the "operability" and "safety". In Table 3, the blending amount of each component and the like are shown, and the results are shown in Table 4.

### [Example 10]

The operation and evaluation were made in the same manner as Example 1 except that the animal body (healthy area, about 30 cm × 30 cm) of livestock (dairy cow) was applied with the base (i) -1 by spraying. In Table 3, the blending amount of each component and the like are shown, and the results are shown in Table 4.

### [Example 11]

The operation and evaluation were made in the same manner as Example 7 except that the animal body (healthy area, about 30 cm × 30 cm) of livestock (dairy cow) was applied with the livestock body protective material by spraying, in the order of the base (i) and (ii) the curing agent. In Table 3, the blending amount of each component and the like are shown, and the results are shown in Table 4.

### [Example 12]

The operation and evaluation were made in the same manner as Example 11 except that the livestock body protective material was applied by spraying in the order of (ii) the curing agent and (i) the base. In Table 3, the blending amount of each component and the like are shown, and the results are shown in Table 4.

### [Comparative Example 1]

The animal body (healthy area) of livestock (dairy cow) was wrapped with a bandage, and evaluation was made in terms of the "operability" and "safety". The results are shown in Table 4.

**[Table 3]**

| | Livestock body protective material (i) Base material / (ii) Curing agent | Subject | Application material | Application method |
|---|---|---|---|---|
| Example 1 | (i)-1 / (ii) None 50 g / 0 g | Animal body of livestock (dairy cow) (Sick/injured area, about 10 cm x 10 cm) | None | Spray |
| Example 2 | (i)-2 / (ii) None 50 g / 0 g | Animal body of livestock (dairy cow) (Sick/injured area, about 10 cm x 10 cm) | None | Spray |
| Example 3 | (i)-3 / (ii) None 50 g / 0 g | Animal body of livestock (dairy cow) (Sick/injured area, about 10 cm x 10 cm) | None | Spray |
| Example 4 | (i)-4 / (ii) None 50 g / 0 g | Animal body of livestock (dairy cow) (Sick/injured area, about 10 cm x 10 cm) | None | Spray |
| Example 5 | (i)-5 / (ii) None 50 g / 0 g | Animal body of livestock (dairy cow) (Sick/injured area, about 10 cm x 10 cm) | None | Spray |
| Example 6 | (i)-1 / (ii) None 75 g / 0 g | Animal body of livestock (dairy cow) (Sick/injured area, about 10 cm x 10 cm) | Gauze | Spray |
| Example 7 | (i)-1 / (ii)-1 50 g / 50 g | Animal body of livestock (dairy cow) (Sick/injured area, about 10 cm x 10 cm) | None | Spray |
| Example 8 | (i)-1 / (ii)-2 50 g / 50 g | Animal body of livestock (dairy cow) (Sick/injured area, about 10 cm x 10 cm) | None | Spray |
| Example 9 | (i)-1 / (ii)-3 50 g / 50 g | Animal body of livestock (dairy cow) (Sick/injured area, about 10 cm x 10 cm) | None | Spray |
| Example 10 | (i)-1 / (ii) None 100 g / 0 g | Animal body of livestock (dairy cow) (Healthy area, about 30 cm x 30 cm) | None | Spray |
| Example 11 | (i)-1 / (ii)-1 100 g / 100 g | Animal body of livestock (dairy cow) (Healthy area, about 30 cm x 30 cm) | None | Spray |
| Example 12 | (i)-1 / (ii)-1 100 g / 100 g | Animal body of livestock (dairy cow) (Healthy area, about 30 cm x 30 cm) | None | Spray |

| | Used material | Subject | Application material | Method |
|---|---|---|---|---|
| Comparative Example 1 | Bandage | Animal body of livestock (dairy cow) (Healthy area) | None | Wrapping |

**[Table 4]**

| | Operability | | | | Safety | |
|---|---|---|---|---|---|---|
| | Time for application work (Seconds) | Time evaluation 1 | Time for peeling work (Seconds) | Time evaluation 2 | Skin removal | Number of bacteria |
| Example 1 | 40 | A | 25 | A | A | - |
| Example 2 | 40 | A | 25 | A | A | - |
| Example 3 | 40 | A | 25 | A | A | - |
| Example 4 | 40 | A | 25 | A | A | - |
| Example 5 | 40 | A | 25 | A | A | - |
| Example 6 | 60 | A | 25 | A | A | - |
| Example 7 | 60 | A | 20 | A | A | - |
| Example 8 | 60 | A | 20 | A | A | - |
| Example 9 | 60 | A | 20 | A | A | - |
| Example 10 | 90 | A | 40 | A | A | A |
| Example 11 | 120 | A | 30 | A | A | A |
| Example 12 | 120 | A | 30 | A | A | A |
| Comparative Example 1 | 480 | C | 300 | C | A | C |

As it is shown in Table 4, in Examples 1 to 12 in which the livestock body protective material was used, a protective film was able to be easily formed in a sick or injured area and also in a healthy area of livestock (dairy cow) within a relatively short time, and also the protective film was able to be easily peeled within a relatively short time. In any of those Examples, a protective film was able to be evenly formed, and also, in any of those Examples, the protective film was able to be peeled all at once. No abnormality was shown from the sick or injured area after the protective film had been removed therefrom, and it was possible to confirm that there was an improvement in injured state. In Example 5, in particular, the injured state has been improved compared to other Examples. In Example 3, since a surfactant was added to the livestock body protective material, more favorable applicability was obtained and peeling was easier compared to other Examples.

On the other hand, in Comparative Example 1 in which a bandage was used instead of the livestock body protective material, it took time even for wrapping a bandage due to wild behavior or the like of livestock. The same problems occurred at the time of removing the bandage. Furthermore, in Comparative Example 1, even if the leg part of livestock was wrapped with a bandage, the bandage was lost or the like as the livestock moved, and thus it was found that the holdability of bandage was very poor. When the livestock having the bandage maintained therewith was observed, the bandage was found to be soiled due to permeation of excretions or muddy water. As a result of performing a bacteria test with those livestock, the number of bacteria has increased in the internal side wrapped with bandage due to the infiltration of excretions or muddy water from the bandage to the inside.

Furthermore, although it is not shown in the table, when a commercially available adhesive was used, time was required for application due to the difficulty in easy stretching. Furthermore, as there was a part which solidified during the application, it was very difficult to have uniform application. As a result, irregularities occurred in the application, and also there was a part that was not able to cover a sick or injured area. Due to those reasons, it is believed that the effect of blocking bacteria is low. Furthermore, since the adhesive was strongly adhered onto a sick or injured area, it was very difficult to peel, and thus the livestock behaved wildly due to pains. Accordingly, only fragmental peeling was yielded without complete peeling. Furthermore, skin on the sick or injured area was also partially peeled off.

Disclosure of Japanese Patent Application No. 2016-117683, filed on June 14, 2016, is incorporated herein in its entirety by reference.

## Claims

1. A livestock body protective material comprising (A) an emulsion obtained by dispersing a rubber-based polymer serving as a dispersoid in a dispersion medium including water as a main component, and that is used to form a protective film for protecting a livestock's sick or injured area.

2. The livestock body protective material according to claim 1, further comprising (B) a coagulant.

3. The livestock body protective material according to claim 2, wherein (B) the coagulant is at least one type selected from calcium salt and magnesium salt.

4. The livestock body protective material according to claim 2, wherein (B) the coagulant is at least one type selected from the group consisting of lactic acid, citric acid, acetic acid, malic acid, fumaric acid, maleic acid, tartaric acid, gluconic acid, succinic acid, propionic acid, and butyric acid.

5. The livestock body protective material according to any one of claims 1 to 4, further comprising at least one type selected from the group consisting of (C) a surfactant, (D) a sterilizing disinfectant, and (E) a pharmaceutical component.

6. A kit for forming a livestock body protective film that is composed of a combination of (i) a base containing (A) an emulsion obtained by dispersing a rubber-based polymer serving as a dispersoid in a dispersion medium including water as a main component, stored in a predetermined container, and (ii) a curing agent containing (B) a coagulant, stored in a container different from the above container,
and that is used to form a protective film for protecting a livestock's sick or injured area.

7. The kit for forming a livestock body protective film according to claim 6, wherein (ii) the curing agent further contains (F) water and/or a water-soluble solvent.

8. A method for protecting a livestock's sick or injured area comprising forming a protective film for protection of a livestock's sick or injured area by adhering the livestock body protective material according to any one of claims 1 to 5 on a livestock's sick or injured area.

9. A method for protecting a livestock's sick or injured area comprising forming a protective film for protection of a livestock's sick or injured area by adhering any one of (i) a base containing (A) an emulsion obtained by dispersing a rubber-based polymer serving as a dispersoid in a dispersion medium including water as a main component and (ii) a curing agent containing (B) a coagulant on a livestock's sick or injured area, and then adhering the other on the livestock's sick or injured area.

10. Use of the livestock body protective material according to any one of claims 1 to 5 or the kit for forming a livestock body protective film according to claim 6 or 7 for forming a protective film for protection of a livestock's sick or injured area.
